# EUROPÄISCHE PATENTSCHRIFT

(11) **EP 1 961 372 B1**
(45) Veröffentlichungstag und Bekanntmachung des Hinweises auf die Patenterteilung: **07.03.2012**
(21) Anmeldenummer: 08150213.0
(22) Anmeldetag: 14.01.2008
(51) Int. Cl.: A61B 1/07

(54) **Endoskop für medizinische Zwecke**
Endoscope for medicinal purposes
Endoscope à des fins médicales

(30) Priorität: 22.02.2007 DE 102007008643
(43) Veröffentlichungstag der Anmeldung: 27.08.2008
(73) Patentinhaber: Aesculap AG, 78532 Tuttlingen (DE)
(72) Erfinder: Weißhaupt, Dieter, 78194, Immendingen (DE)
(74) Vertreter: Hoeger, Stellrecht & Partner Patentanwälte

(56) Entgegenhaltungen:
- US-A- 4 580 198
- US-A- 5 924 978
- US-A1- 2002 022 763

## Beschreibung

Die Erfindung betrifft ein Endoskop für medizinische Zwecke mit einem Rohrschaft, der an seinem distalen Ende eine Betrachtungsöffnung aufweist, und mit einem Lichtleiter in dem Rohrschaft, der von einem Lichtleiteranschluss am proximalen Ende des Rohrschaftes zu einer Lichtaustrittsstelle am distalen Ende des Rohrschaftes verläuft.

Endoskope dieser Art werden in der Chirurgie allgemein eingesetzt, insbesondere im Bereich der minimalinvasiven Chirurgie. Zur Beleuchtung der betrachteten Eingriffsstelle ist es bekannt, das Endoskop am Lichtleiteranschluss mit einem Lichtleiter zu verbinden, der in der Regel mit einer stationären Lichtquelle verbunden ist und auf diese Weise Licht über den Lichtleiteranschluss und durch den im Rohrschaft angeordneten Lichtleiter zur Lichtaustrittsstelle transportiert.

Die Verbindung des Endoskopes mit einer stationären Lichtquelle über ein am Endoskop angeschlossenes Lichtleiterkabel ist bei der Handhabung des Endoskopes teilweise störend, und daher liegt der Erfindung die Aufgabe zugrunde, ein Endoskop der gattungsgemäßen Art so auszubilden, dass die Handhabbarkeit des Endoskopes verbessert wird.

Diese Aufgabe wird bei einem Endoskop der eingangs beschriebenen Art erfindungsgemäß durch die Merkmale von Anspruch 1 gelöst.

Auf diese Weise ist das Endoskop vollständig unabhängig von einer stationären Leuchtquelle, und es ist ein großer Vorteil, dass für den Anschluss der transportablen Lichtquelle der ohnehin vorgesehene Lichtleiteranschluss verwendet werden kann, an den anstelle des üblichen Lichtleiterkabels nunmehr die transportable Lichtquelle mit der Batterie und der Leuchtdiode angeschlossen wird, wobei allein durch das Anschließen an den Lichtleiteranschluss der die Leuchtdiode mit der Batterie verbindende Schalter geschlossen wird. Der Operateur muss also lediglich bei der Inbetriebnahme des Endoskopes die Lichtquelle an den Lichtleiteranschluss anschließen, und dann liefert diese über den im Rohrschaft angeordneten Lichtleiter die gewünschte Beleuchtung im Bereich der Lichtaustrittsstelle.

Insbesondere kann vorgesehen sein, dass die Lichtquelle auf den Lichtleiteranschluss aufgeschraubt ist und der Schalter bei der Aufschraubbewegung geschlossen wird. Bei der Aufschraubbewegung wird die Lichtquelle auch in axialer Richtung verschoben, und diese Verschiebung in axialer Richtung kann ausgenutzt werden, um den Schalter zu schließen.

Bei einer bevorzugten Ausführungsform ist vorgesehen, dass die Lichtquelle ein Gehäuse aufweist, in dem die Batterie und die Leuchtdiode angeordnet sind.

Besonders vorteilhaft ist es, wenn die Leuchtdiode und/oder die Batterie in dem Gehäuse zwischen einem anschlussseitigen und einem anschlussabgewandten Ende längsverschieblich gelagert sind und durch eine Feder gegen das anschlussseitige Ende des Gehäuses gedrückt werden.

Insbesondere ist dabei eine Ausgestaltung günstig, bei der der Lichtleiteranschluss beim Anschließen der Lichtquelle in das Gehäuse eindringt und dabei die Leuchtdiode und/oder die Batterie in das Gehäuse einschiebt und dadurch den Stromkreis zur Versorgung der Leuchtdiode durch die Batterie schließt.

Es kann vorgesehen sein, dass der Schalter zwei Kontakte aufweist, von denen einer fest am Gehäuse angeordnet ist und der andere an der verschieblich gelagerten Leuchtdiode oder der verschieblich gelagerten Batterie.

Erfindungsgemäß ist vorgesehen, dass der Schalter nach dem Schließen nicht aus seiner Schließstellung in eine Offenstellung zurückschaltbar ist. Die Lichtquelle ist also nach einem einmaligen Einsatz verbraucht und muss entsorgt werden, sei es, dass sie als Wegwerfteil ausgebildet ist, sei es, dass sie mit neuen Batterien bestückt werden muss.

Zu diesem Zweck ist vorgesehen , dass bei dem Schalter, der durch die Verschiebung der Leuchtdiode und/oder der Batterie beim Anschließen der Lichtquelle an den Lichtleiteranschluss geschlossen wird, eine die Rückbewegung der Leuchtdiode und/oder der Batterie verhindernde Blockiereinrichtung vorhanden ist.

Die Blockiereinrichtung kann beispielsweise einen federnden Riegel aufweisen, der beim Verschieben der Leuchtdiode und/oder der Batterie in eine Ausnehmung der Lichtquelle federnd eintaucht und dadurch eine Rückbewegung der Leuchtdiode und/oder der Batterie verhindert.

Bei einer anderen Ausführungsform ist vorgesehen, dass die Blockiereinrichtung einen radial elastisch zusammendrückbaren Sprengring aufweist, der beim Verschieben der Leuchtdiode und/oder der Batterie in eine Ausnehmung der Lichtquelle federnd eintaucht und dadurch eine Rückbewegung der Leuchtdiode und/oder der Batterie verhindert.

Bei einer noch anderen Ausgestaltung ist vorgesehen, dass die Blockiereinrichtung Vor- und Rücksprünge an dem Gehäuse einerseits und der Leuchtdiode und/oder der Batterie andererseits umfasst, die beim Verschieben der Leuchtdiode und/oder der Batterie elastisch oder plastisch verformt werden und dadurch eine Rückbewegung der Leuchtdiode und/oder der Batterie verhindern.

Vorzugsweise ist das Gehäuse zylinderförmig ausgebildet, so dass es auf den rohrstutzenförmigen Lichtleiteranschluss aufgeschraubt werden kann und vorzugsweise einen Außendurchmesser aufweist, der dem des Lichtleiteranschlusses entspricht. Es ergibt sich dadurch ein insgesamt sehr handlicher Aufbau des Endoskops.

Bei einer bevorzugten Ausführungsform sind die Leuchtdiode und die Batterie in einer Aufnahmehülse angeordnet, die im Gehäuse längsverschieblich gelagert ist.

Diese Aufnahmehülse kann insbesondere eine Kontaktfeder tragen, die beim Einschieben der Aufnahmehülse in das Gehäuse an einem Kontakt des Gehäuses zur Anlage kommt und dadurch den Stromkreis zur Versorgung der Leuchtdiode schließt.

Um die Unabhängigkeit des Endoskops von stationären Energieversorgungen und Kabeln weiter zu verbessern, kann zusätzlich vorgesehen sein, dass auf ein Okular des Endoskops eine elektronische Kamera aufgesetzt ist, vorzugsweise durch elastisches Aufschnappen. Wenn diese elektronische Kamera gemäß einer Weiterbildung der Erfindung eine Einrichtung zur drahtlosen Signalübertragung aufweist, kann das von der Kamera aufgenommene Bild drahtlos an eine Empfangseinrichtung übertragen werden, d.h. das Endoskop kann ohne jegliche Kabelverbindung betrieben werden und trotzdem einerseits eine Bearbeitungsstelle beleuchten und andererseits das betrachtete Bild dieser Eingriffsstelle über die Kamera an die externe Beobachtungseinrichtung übertragen.

US 4-580-198-A und US-5-924-978-A zeigen jeweils ein medizinisches Endoskop mit einem Lichtleiter und einem Schalter, der eine Lichtquelle beim Anschließen an den Lichtleiteranschluss zwangsläufig mit einer Batterie verbindet.

Die nachfolgende Beschreibung bevorzugter Ausführungsformen der Erfindung dient im Zusammenhang mit der Zeichnung der näheren Erläuterung. Es zeigen:

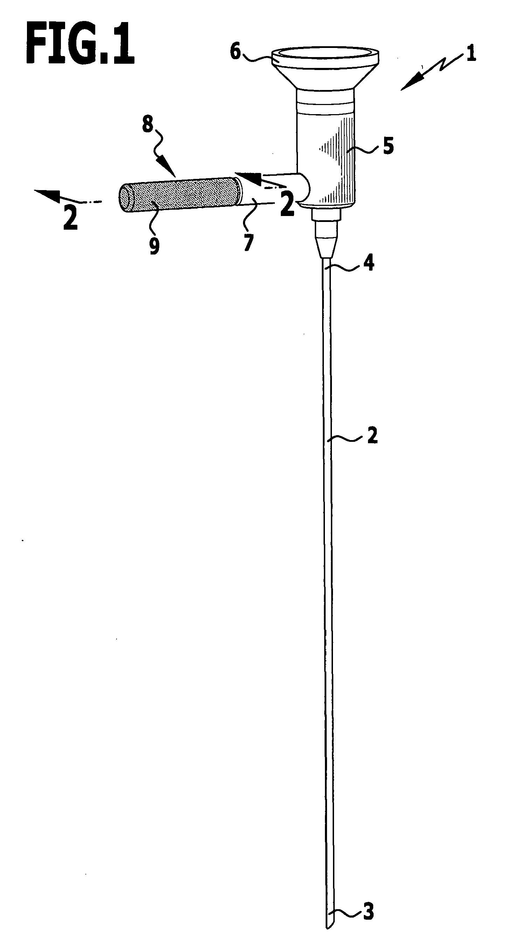

- Figur 1:: eine perspektivische Ansicht eines Endoskops mit einer auf den Lichtleiteranschluss aufgeschraubten Lichtquelle;
- Figur 2:: eine Schnittansicht längs Linie 2-2 in Figur 1 vor dem vollständigen Aufschrauben der Lichtquelle auf den Lichtleiteranschluss;
- Figur 3:: eine Ansicht ähnlich Figur 2 nach dem vollständigen Aufschrauben der Lichtquelle auf den Lichtleiteranschluss;
- Figur 4:: eine Ansicht ähnlich Figur 2 mit einer Blockiereinrichtung in Form von verformbaren Vorsprüngen;
- Figur 5:: eine Ansicht des Bereiches A in Figur 4 vor dem vollständigen Aufschrauben der Lichtquelle auf den Lichtleiteranschluss;
- Figur 6:: eine Ansicht ähnlich Figur 5 nach dem vollständigen Aufschrauben der Lichtquelle auf den Lichtleiteranschluss;
- Figur 7:: eine Ansicht ähnlich Figur 4 mit einer Blockiereinrichtung in Form eines Sprengringes;
- Figur 8:: eine vergrößerte Darstellung des Bereiches B in Figur 7 vor dem vollständigen Aufschrauben der Lichtquelle auf den Lichtleiteranschluss;
- Figur 9:: eine Ansicht ähnlich Figur 8 nach dem vollständigen Aufschrauben der Lichtquelle auf den Lichtleiteranschluss und
- Figur 10:: eine Seitenansicht eines Endoskops mit einer auf den Lichtleiteranschluss aufgeschraubten Lichtquelle und mit einer auf das Okular aufgeschnappten, drahtlos übertragenden elektronischen Kamera.

Das in der Zeichnung dargestellte Endoskop 1 umfasst einen länglichen Rohrschaft 2 mit einem distalen Ende 3 und einem proximalen Ende 4, an das proximale Ende 4 schließt sich ein Anschlussteil 5 mit einem Okular 6 und einem seitlich vom Anschlussteil 5 abstehenden Lichtleiteranschluss 7 an. Von dem Lichtleiteranschluss, der als Schraubanschluss ausgeführt ist, führt im Inneren des Rohrschaftes 2 ein in der Zeichnung nicht dargestelltes Lichtleiterkabel bis zu einer am distalen Ende 3 des Rohrschaftes 2 angeordneten Lichtaustrittsstelle, so dass vor dem distalen Ende 3 ein Operationsfeld beleuchtet werden kann, wenn an dem Lichtleiteranschluss 7 Licht eingespeist wird.

Außerdem befindet sich in dem Rohrschaft 2 in aus der Zeichnung nicht ersichtlicher und an sich bekannter Weise eine Optik, die es ermöglicht, das vor dem distalen Ende 3 des Rohrschaftes 2 angeordnete Operationsgebiet durch das Okular 6 zu betrachten.

Zur Beleuchtung des Operationsgebietes kann an den Lichtleiteranschluss 7 eine Lichtquelle 8 angeschlossen werden, und zwar eine transportable, kabellose Lichtquelle. Diese Lichtquelle weist ein zylindrisches Gehäuse 9 auf, dessen Außendurchmesser im Wesentlichen dem Außendurchmesser des Lichtleiteranschlusses 7 entspricht, und das Gehäuse 9 ist auf den Lichtleiteranschluss 7 aufschraubbar ausgebildet.

Dazu weist das Gehäuse 9 an seiner dem Lichtleiteranschluss 7 zugewandten Seite eine Innengewindebohrung 10 auf, auf der gegenüberliegenden Seite ist das Gehäuse 9 durch einen Gewindestopfen 11 verschlossen, der in eine an dieser Seite angeordnete Innengewindebohrung 12 des Gehäuses 9 eingeschraubt ist.

Im Inneren des Gehäuses 9 ist eine Aufnahmehülse 13 in Längsrichtung verschieblich angeordnet, in der hintereinander eine Leuchtdiode 14 und eine aus mehreren Zellen 15 bestehende Batterie 16 angeordnet sind. An der Batterie 16 stützt sich eine Schraubenfeder 17 ab, die mit ihrem gegenüberliegenden Ende an dem Gewindestopfen 11 anliegt und dadurch die Aufnahmehülse 13 in Richtung auf die Innengewindebohrung 10 verschiebt, also in Richtung auf den Lichtleiteranschluss 7. Die Leuchtdiode 14 ragt an der Seite der Innengewindebohrung 10 aus der Aufnahmehülse 13 hervor und taucht in den Lichtleiteranschluss 7 geringfügig ein.

An der Außenseite der Aufnahmehülse 13 ist ein sich über dessen gesamte Länge erstreckender Kontaktstreifen 18 angeordnet, der einerseits mit der Leuchtdiode 14 verbunden ist und andererseits geringfügig über das der Leuchtdiode abgewandte Ende der Batterie 16 hervorsteht. Dieser Kontaktstreifen 18 ist ein Kontakt eines Schalters, dessen anderer Kontakt durch den Gewindestopfen 11 gebildet wird, der über die Schraubenfeder 17 elektrisch leitend mit einem Ende der Batterie 16 verbunden ist, während das andere Ende der Batterie mit einem zweiten Pol der Leuchtdiode 14 verbunden ist.

Vor dem Aufschrauben der Lichtquelle 8 auf den Lichtleiteranschluss verschiebt die Schraubenfeder 17 die Aufnahmehülse 13 mit der darin angeordneten Leuchtdiode 14 und der darin angeordneten Batterie 16 so in Richtung auf die Innengewindebohrung 10, dass der Kontaktstreifen 18 von dem Gewindestopfen 11 einen Abstand einhält, der Schalter ist damit nicht geschlossen und damit kann kein Strom durch die Leuchtdiode fließen.

Beim Aufschrauben der Lichtquelle 8 auf den Lichtleiteranschluss 7 tritt dieser durch die Innengewindebohrung 10 in das Innere des Gehäuses 9 ein und verschiebt dabei die Aufnahmehülse 13 entgegen der Wirkung der Schraubenfeder 17, bis der Kontaktstreifen 18 an dem Gewindestopfen 11 anliegt und damit den Schalter schließt, d.h. die Leuchtdiode 14 wird durch die Batterie 16 mit Energie versorgt und leuchtet. Das von der Leuchtdiode emittierte Licht wird über den Lichtleiter im Rohrschaft 2 an das distale Ende 3 des Rohrschaftes 2 geleitet und dort abgegeben.

Allein durch das Aufschrauben der Lichtquelle 8 auf den Lichtleiteranschluss 7 wird somit die Leuchtdiode eingeschaltet.

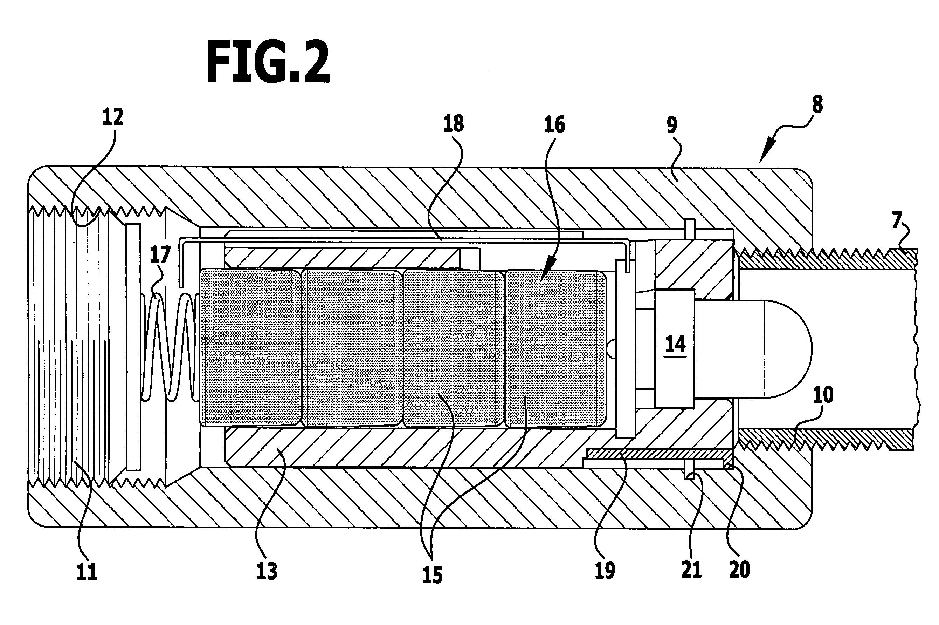

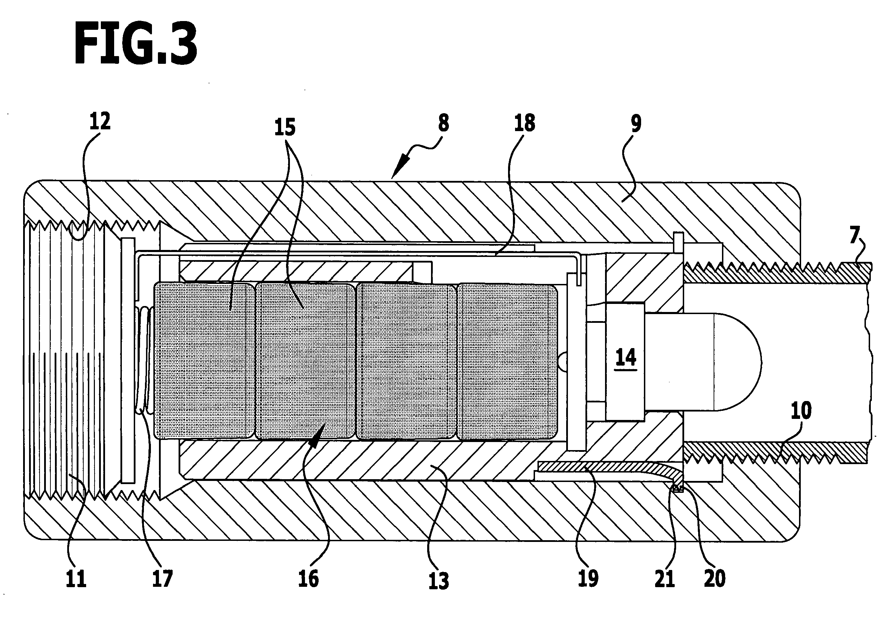

Bei dem in den Figuren 2 und 3 dargestellten Ausführungsbeispiel ist in den Außenmantel der Aufnahmehülse 13 ein elastischer Riegel 19 eingelegt, mit einem radial abstehenden Ende 20, das sich an der Innenwand des Gehäuses 9 abstützt. In dieser Innenwand ist eine Umfangsnut 21 eingelassen, in die das Ende 20 des federnden Riegels 19 federnd eintaucht, wenn dieses Ende 20 bei der Verschiebung der Aufnahmehülse 13 über die Umfangsnut 21 gleitet. Dadurch wird die Aufnahmehülse 13 gegen eine Verschiebung in beiden Richtungen gesichert, und dies führt dazu, dass der Schalter eingeschaltet bleibt, auch wenn die Lichtquelle 8 wieder von dem Lichtleiteranschluss 7 abgeschraubt wird. Man erhält auf diese Weise also einen Schalter, der zwar eingeschaltet, nicht aber ausgeschaltet werden kann.

Dadurch wird sichergestellt, dass die Lichtquelle 8 nicht erneut an einem anderen Endoskop 1 eingesetzt wird, sondern es ist notwendig, die Lichtquelle 8 entweder wegzuwerfen oder aber durch Auswechseln der Batterie und durch Sterilisation wieder in den Gebrauchszustand zu überführen.

Bei dem in den Figuren 2 und 3 dargestellten Ausführungsbeispiel wird die Festlegung des Schalters in der eingeschalteten Position durch den Riegel 19 gewährleistet.

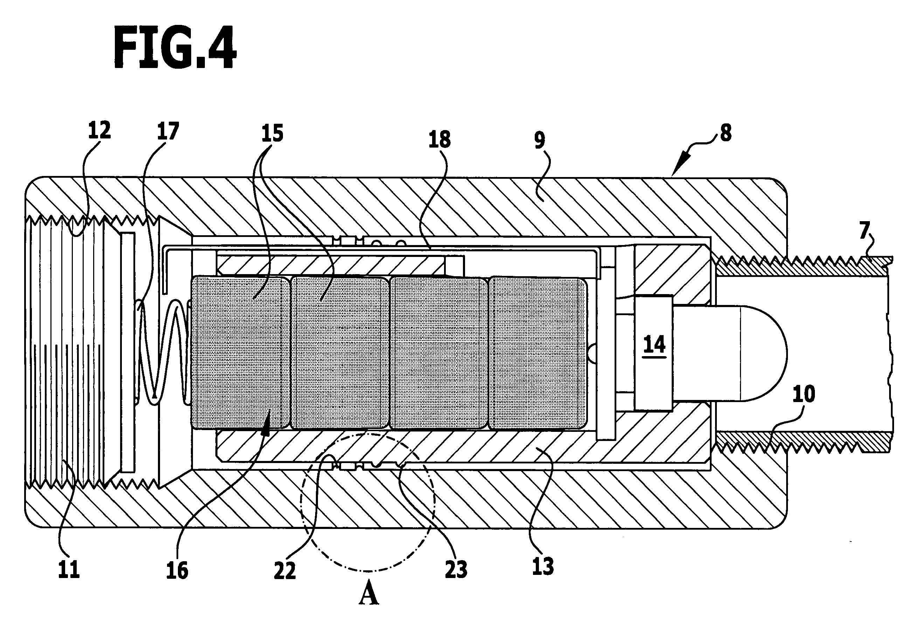

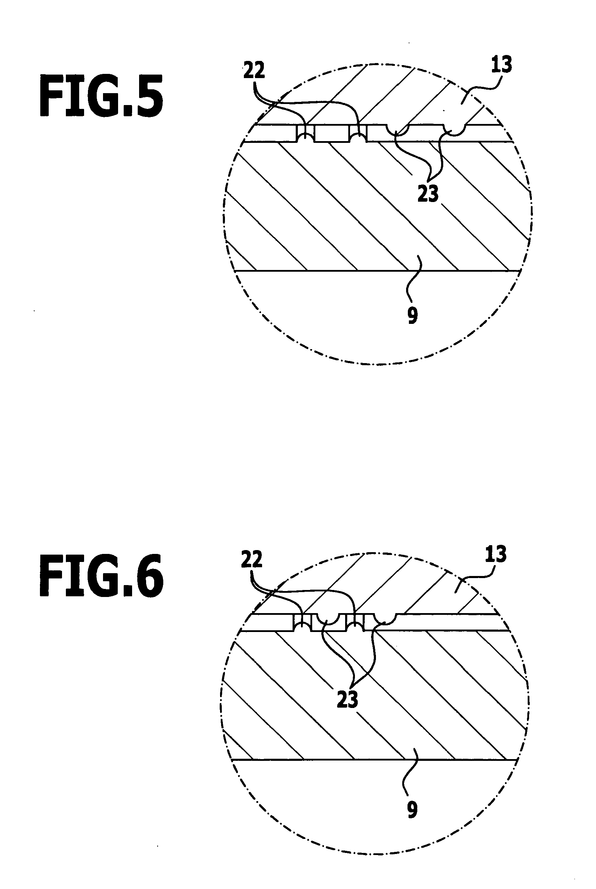

Bei einem abgewandelten Ausführungsbeispiel, wie es in den Figuren 4 bis 6 dargestellt ist, tragen das Gehäuse 9 an seiner Innenseite und die Aufnahmehülse 13 an ihrer Außenseite Vorsprünge 22 bzw. 23, beispielsweise in Form von umlaufenden Rippen oder Schultern, die beim Verschieben der Aufnahmehülse 13 aneinander vorbei gleiten und dann entweder elastisch oder plastisch verformt werden. Dies führt dazu, dass die Aufnahmehülse 13 zwar in einer Richtung verschoben werden kann, weil die Verschiebekraft beim Aufschrauben der Lichtquelle 8 auf den Lichtleiteranschluss 7 groß ist. Die Rückstellkraft der Schraubenfeder 17 reicht aber nicht aus, um die Aufnahmehülse 13 in der umgekehrten Richtung an den Vorsprüngen vorbei zu verschieben, so dass der Schalter aktiviert bleibt.

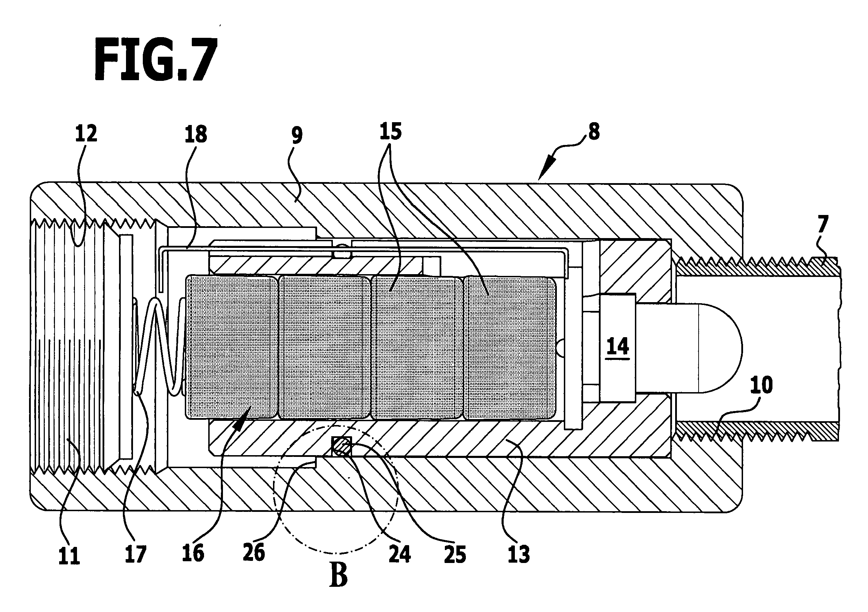

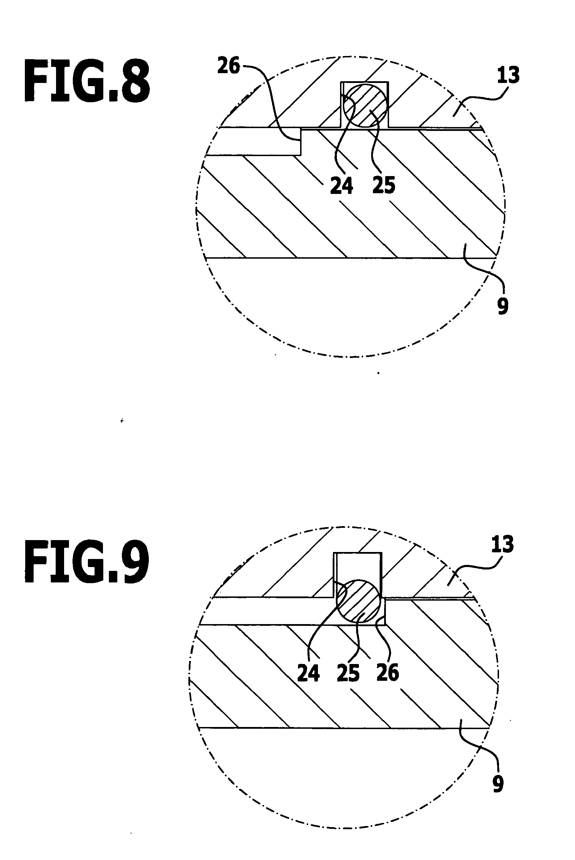

Bei dem Ausführungsbeispiel der Figuren 7 bis 9 ist in eine Umfangsnut 24 der Aufnahmehülse 13 ein Sprengring 25 eingelegt, der beim Verschieben der Aufnahmehülse an einer Stufe 26 an der Innenwand des Gehäuses 9 vorbei gleitet und sich dadurch elastisch ausdehnt, so dass ein Rückschieben der Aufnahmehülse 13 verhindert wird.

In allen Fällen ist also eine Blockiereinrichtung vorgesehen, die zwar die Verschiebung der Aufnahmehülse in einen Einschaltzustand ermöglicht, nicht aber das Zurückschieben in Ausschaltrichtung.

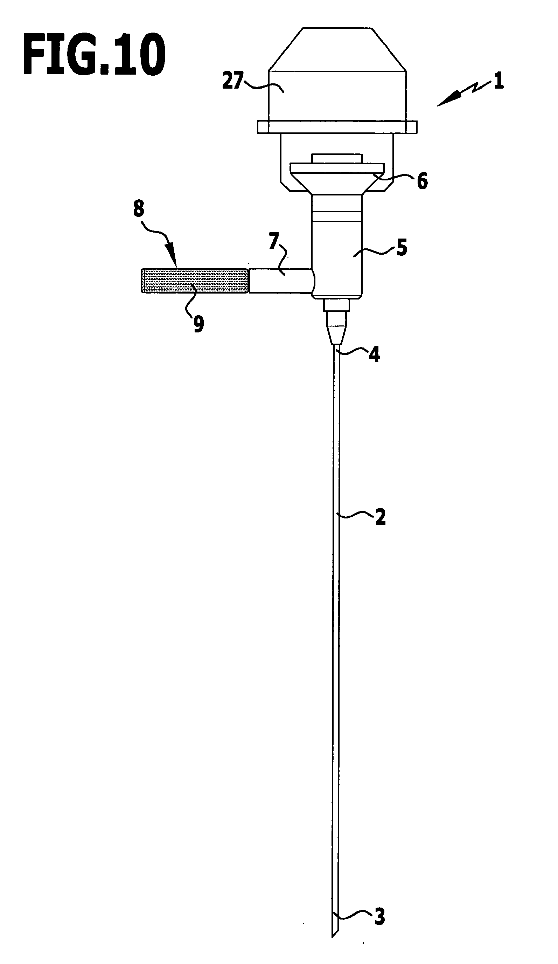

Aus der Darstellung der Figur 10 wird deutlich, dass auf das Okular 6 eine elektronische Festkörperkamera 27 elastisch aufgeschnappt werden kann, die das durch das Okular 6 empfangene Bild durch eine geeignete Elektronik drahtlos an eine in der Zeichnung nicht dargestellte externe Empfangseinrichtung übertragen kann, so dass das Endoskop 1 ohne Kabel für die Beleuchtung und ohne Kabel für die Betrachtung frei gehandhabt werden kann.

Die beschriebene Lichtquelle und auch die beschriebene Festkörperkamera können auf Endoskope herkömmlicher Art nachträglich aufgesetzt und von diesen auch wieder abgenommen werden, da die Dimensionierung der Innengewindebohrung 10 den üblicherweise verwendeten Dimensionen der Lichtleiteranschlüsse 7 bei Endoskopen entspricht und da die Festkörperkamera 27 lösbar auf das Okular aufgeclipst ist.

## Patentansprüche

1. Endoskop (1) für medizinische Zwecke mit einem Rohrschaft (2), der an seinem distalen Ende (3) eine Betrachtungsöffnung aufweist, und mit einem Lichtleiter in dem Rohrschaft (2), der von einem Lichtleiteranschluss (7) am proximalen Ende (4) des Rohrschaftes (2) zu einer Lichtaustrittsstelle am distalen Ende (3) des Rohrschaftes (2) verläuft, wobei an den Lichtleiteranschluss (7) eine Lichtquelle (8) mit einer Batterie (16) und einer Leuchtdiode (14) angeschlossen ist, die einen Schalter (18, 11) aufweist, der die Leuchtdiode (14) beim Anschließen der Lichtquelle (8) an dem Lichtleiteranschluss (7) zwangsläufig mit der Batterie (16) verbindet, **dadurch gekennzeichnet, dass** bei dem Schalter, der durch die Verschiebung der Leuchtdiode (14) und/oder der Batterie (16) beim Anschließen der Lichtquelle (8) an den Lichtleiteranschluss (7) geschlossen wird, eine die Rückbewegung der Leuchtdiode (14) und/oder der Batterie (16) verhindernde Blockiereinrichtung vorgesehen ist, so dass der Schalter nach dem Schließen nicht aus einer Schließstellung in eine Offenstellung zurückschaltbar ist.

2. Endoskop nach Anspruch 1, **dadurch gekennzeichnet, dass** die Lichtquelle (8) auf den Lichtleiteranschluss (7) aufgeschraubt ist und der Schalter bei der Aufschraubbewegung geschlossen wird.

3. Endoskop nach einem der Ansprüche 1 oder 2, **dadurch gekennzeichnet, dass** die Lichtquelle (8) ein Gehäuse (9) aufweist, in dem die Batterie (16) und die Leuchtdiode (14) angeordnet sind.

4. Endoskop nach Anspruch 3, **dadurch gekennzeichnet, dass** die Leuchtdiode (14) und/oder die Batterie (16) in dem Gehäuse (9) zwischen einem anschlussseitigen und einem anschlussabgewandten Ende längsverschieblich gelagert sind und durch eine Feder (17) gegen das anschlussseitige Ende des Gehäuses (9) gedrückt werden.

5. Endoskop nach Anspruch 4, **dadurch gekennzeichnet, dass** der Lichtleiteranschluss (7) beim Anschließen der Lichtquelle (8) in das Gehäuse (9) eindringt und dabei die Leuchtdiode (14) und/oder die Batterie (16) in das Gehäuse (9) einschiebt und **dadurch** den Stromkreis zur Versorgung der Leuchtdiode (14) durch die Batterie (16) schließt.

6. Endoskop nach Anspruch 5, **dadurch gekennzeichnet, dass** der Schalter zwei Kontakte (18, 11) aufweist, von denen einer fest am Gehäuse (9) angeordnet ist und der andere an der verschieblich gelagerten Leuchtdiode (14) oder der verschieblich gelagerten Batterie (16).

7. Endoskop nach einem der voranstehenden Ansprüche, **dadurch gekennzeichnet, dass** die Blockiereinrichtung einen federnden Riegel (19) aufweist, der beim Verschieben der Leuchtdiode (14) und/oder der Batterie (16) in eine Ausnehmung (21) der Lichtquelle (8) federnd eintaucht und **dadurch** eine Rückbewegung der Leuchtdiode (14) und/oder der Batterie (16) verhindert.

8. Endoskop nach einem der Ansprüche 1 bis 6, **dadurch gekennzeichnet, dass** die Blockiereinrichtung einen radial elastisch zusammendrückbaren Sprengring (25) aufweist, der beim Verschieben der Leuchtdiode (14) und/oder der Batterie (16) in eine Ausnehmung (26) der Lichtquelle (8) federnd eintaucht und **dadurch** eine Rückbewegung der Leuchtdiode (14) und/oder der Batterie (16) verhindert.

9. Endoskop nach einem der Ansprüche 1 bis 6, **dadurch gekennzeichnet, dass** die Blockiereinrichtung Vor- und Rücksprünge (22, 23) an dem Gehäuse (9) einerseits und der Leuchtdiode (14) und/oder der Batterie (16) andererseits umfasst, die beim Verschieben der Leuchtdiode (14) und/oder der Batterie (16) elastisch oder plastisch verformt werden und **dadurch** eine Rückbewegung der Leuchtdiode (14) und/oder der Batterie (16) verhindern.

10. Endoskop nach einem der Ansprüche 3 bis 9, **dadurch gekennzeichnet, dass** das Gehäuse (9) zylinderförmig ausgebildet ist.

11. Endoskop nach einem der Ansprüche 3 bis 10, **dadurch gekennzeichnet, dass** die Leuchtdiode (14) und die Batterie (16) in einer Aufnahmehülse (13) angeordnet sind, die im Gehäuse (9) längsverschieblich gelagert ist.

12. Endoskop nach Anspruch 11, **dadurch gekennzeichnet, dass** die Aufnahmehülse (13) eine Kontaktfeder (18) trägt, die beim Einschieben der Aufnahmehülse (13) in das Gehäuse (9) an einem Kontakt (11) des Gehäuses (9) zur Anlage kommt und **dadurch** den Stromkreis zur Versorgung der Leuchtdiode (14) schließt.

13. Endoskop nach einem der voranstehenden Ansprüche, **dadurch gekennzeichnet, dass** auf ein Okular (6) des Endoskops (1) eine elektronische Kamera (27) aufgesetzt ist.

14. Endoskop nach Anspruch 13, **dadurch gekennzeichnet, dass** die elektronische Kamera (27) lösbar auf das Okular (6) aufgeschnappt ist.

15. Endoskop nach Anspruch 13 oder 14, **dadurch gekennzeichnet, dass** die elektronische Kamera (27) eine Einrichtung zur drahtlosen Signalübertragung aufweist.

## Claims

1. Endoscope (1) for medical purposes with a tubular shaft (2), which has an observation opening at its distal end (3), and with a light conductor in the tubular shaft (2), which runs from a light conductor connection (7) at the proximal end (4) of the tubular shaft (2) to a light output point at the distal end (3) of the tubular shaft (2), wherein connected to the light conductor connection (7) is a light source (8) with a battery (16) and a light-emitting diode (14), which has a switch (18, 11), which positively connects the light-emitting diode (14) to the battery (16) when the light source (8) is connected to the light conductor connection (7), **characterised in that** in the switch, which is closed by the displacement of the light-emitting diode (14) and /or the battery (16) when the light source (8) is connected to the light conductor connection (7), there is provided a blocking device preventing the return movement of the light-emitting diode (14) and/or the battery (16) so that after closing, the switch is not switchable back from a closing position into an open position.

2. Endoscope according to claim 1, **characterised in that** the light source (8) is screwed onto the light conductor connection (7) and the switch is closed during the screwing-on movement.

3. Endoscope according to either of claims 1 or 2, **characterised in that** the light source (8) has a housing (9), in which the battery (16) and the light-emitting diode (14) are arranged.

4. Endoscope according to claim 3, **characterised in that** the light-emitting diode (14) and/or the battery (16) are longitudinally displaceably mounted in the housing (9) between an end on the connection side and an end remote from the connection and are pressed by a spring (17) against the end of the housing (9) on the connection side.

5. Endoscope according to claim 4, **characterised in that** the light conductor connection (7) enters the housing (9) when the light source (8) is connected and in the process pushes the light-emitting diode (14) and/or the battery (16) into the housing (9) and thereby closes the electric circuit to supply the light-emitting diode (14) by means of the battery (16).

6. Endoscope according to claim 5, **characterised in that** the switch has two contacts (18, 11), one of which is fixedly arranged on the housing (9) and the other on the displaceably mounted light-emitting diode (14) or the displaceably mounted battery (16).

7. Endoscope according to any one of the preceding claims, **characterised in that** the blocking device has a resilient latch (19), which, when the light-emitting diode (14) and/or the battery (16) are displaced, enters a recess (21) of the light source (8) in a resilient manner and thereby prevents a return movement of the light-emitting diode (14) and/or the battery (16).

8. Endoscope according to any one of claims 1 to 6, **characterised in that** the blocking device has a radially elastically compressible snap ring (25), which, upon the displacement of the light-emitting diode (14) and/or the battery (16), enters a recess (26) of the light source (8) in a resilient manner and thereby prevents a return movement of the light-emitting diode (14) and/or the battery (16).

9. Endoscope according to any one of claims 1 to 6, **characterised in that** the blocking device comprises projections and recesses (22, 23) on the housing (9), on the one hand, and on the light-emitting diode (14) and/or the battery (16), on the other hand, which are elastically or plastically deformed upon displacement of the light-emitting diode (14) and/or the battery (16) and thereby prevent a return movement of the light-emitting diode (14) and/or the battery (16).

10. Endoscope according to any one of claims 3 to 9, **characterised in that** the housing (9) is cylindrical.

11. Endoscope according to any one of claims 3 to 10, **characterised in that** the light-emitting diode (14) and the battery (16) are arranged in a receiving sleeve (13), which is longitudinally displaceably mounted in the housing (9).

12. Endoscope according to claim 11, **characterised in that** the receiving sleeve (13) carries a contact spring (18), which, on insertion of the receiving sleeve (13) into the housing (9), abuts a contact (11) of the housing (9) and thereby closes the electric circuit to supply the light-emitting diode (14).

13. Endoscope according to any one of the preceding claims, **characterised in that** an electronic camera (27) is placed on an eyepiece (6) of the endoscope (1).

14. Endoscope according to claim 13, **characterised in that** the electronic camera (27) is detachably snapped onto the eyepiece (6).

15. Endoscope according to claim 13 or 14, **characterised in that** the electronic camera (27) has a device for wireless signal transmission.

## Revendications

1. Endoscope (1) destiné à des fins médicales, comprenant un corps allongé tubulaire (2), qui présente une ouverture d'observation à son extrémité distale (3), et comprenant également dans le corps allongé tubulaire (2), une fibre optique qui s'étend d'un raccord de branchement de fibre optique (7) au niveau de l'extrémité proximale (4) du corps allongé tubulaire (2) jusqu'à une zone de sortie de lumière à l'extrémité distale (3) du corps allongé tubulaire (2), une source de lumière (8) avec une batterie (16) et une diode électroluminescente (14) étant raccordée au raccord de branchement de fibre optique (7) et comportant un interrupteur (18, 11) qui, lors du branchement de la source de lumière (8) au raccord de branchement de fibre optique (7), relie obligatoirement la diode électroluminescente (14) à la batterie (16), **caractérisé en ce que** concernant l'interrupteur qui se ferme suite au coulissement de la diode électroluminescente (14) et/ou de la batterie (16) lors du raccordement de la source de lumière (8) au raccord de branchement de fibre optique (7), il est prévu un mécanisme de blocage empêchant un mouvement de retour de la diode électroluminescente (14) et/ou de la batterie (16), de sorte que l'interrupteur, après sa fermeture, ne peut pas être commuté en retour de sa position fermée à une position ouverte.

2. Endoscope selon la revendication 1, **caractérisé en ce que** la source de lumière (8) est montée par vissage sur le raccord de branchement de fibre optique (7), et l'interrupteur se ferme au cours du mouvement de montage par vissage.

3. Endoscope selon l'une des revendications 1 ou 2, **caractérisé en ce que** la source de lumière (8) présente un boitier (9) dans lequel sont agencées la batterie (16) et la diode électroluminescente (14).

4. Endoscope selon la revendication 3, **caractérisé en ce que** la diode électroluminescente (14) et/ou la batterie (16) sont montées dans le boitier (9) de manière à pouvoir coulisser longitudinalement entre une extrémité côté raccordement et une extrémité opposée à celle du côté raccordement, et sont pressées par un ressort (17) contre l'extrémité côté raccordement du boitier (9).

5. Endoscope selon la revendication 4, **caractérisé en ce que** lors du raccordement de la source de lumière (8), le raccord de branchement de fibre optique (7) pénètre dans le boitier (9) et repousse à cette occasion la diode électroluminescente (14) et/ou la batterie (16) dans le boitier (9), en produisant ainsi la fermeture du circuit de courant pour l'alimentation de la diode électroluminescente (14) par la batterie (16).

6. Endoscope selon la revendication 5, **caractérisé en ce que** l'interrupteur comporte deux contacts (18, 11) dont l'un est agencé de manière fixe sur le boitier (9), et l'autre sur la diode électroluminescente (14) montée coulissante ou sur la batterie (16) montée coulissante.

7. Endoscope selon l'une des revendications précédentes, **caractérisé en ce que** le mécanisme de blocage comprend un verrou élastique (19), qui, lors du coulissement de la diode électroluminescente (14) et/ou de la batterie (16), s'engage de manière élastique dans un évidement (21) de la source de lumière (8), en empêchant ainsi un mouvement de retour de la diode électroluminescente (14) et/ou de la batterie (16).

8. Endoscope selon l'une des revendications 1 à 6, **caractérisé en ce que** le mécanisme de blocage comprend un jonc (25) pouvant être compressé radialement de manière élastique, qui, lors du coulissement de la diode électroluminescente (14) et/ou de la batterie (16), s'engage de manière élastique dans un évidement (26) de la source de lumière (8), en empêchant ainsi un mouvement de retour de la diode électroluminescente (14) et/ou de la batterie (16).

9. Endoscope selon l'une des revendications 1 à 6, **caractérisé en ce que** le mécanisme de blocage comprend des protubérances et des retraits (22, 23) sur le boitier (9) d'une part et sur la diode électroluminescente (14) et/ou la batterie (16) d'autre part, qui, lors du coulissement de la diode électroluminescente (14) et/ou de la batterie (16), sont déformés de manière élastique ou plastique, en empêchant ainsi un mouvement de retour de la diode électroluminescente (14) et/ou de la batterie (16).

10. Endoscope selon l'une des revendications 3 à 9, **caractérisé en ce que** le boitier (9) est d'une configuration de forme cylindrique.

11. Endoscope selon l'une des revendications 3 à 10, **caractérisé en ce que** la diode électroluminescente (14) et la batterie (16) sont agencées dans une douille de réception (13), qui est montée en coulissement longitudinal dans le boitier (9).

12. Endoscope selon la revendication 11, **caractérisé en ce que** la douille de réception (13) porte un ressort de contact (18), qui, lors de l'insertion par coulissement de la douille de réception (13) dans le boitier (9), vient s'appliquer contre un contact (11) du boitier (9), en fermant ainsi le circuit de courant pour l'alimentation de la diode électroluminescente (14).

13. Endoscope selon l'une des revendications précédentes, **caractérisé en ce qu'**une caméra électronique (27) est rapportée sur un oculaire (6) de l'endoscope (1).

14. Endoscope selon la revendication 13, **caractérisé en ce que** la caméra électronique (27) est enclenchée de manière amovible sur l'oculaire (6).

15. Endoscope selon la revendication 13 ou la revendication 14, **caractérisé en ce que** la caméra électronique (27) comporte un dispositif pour la transmission de signal, sans fil.
